(19) Europäisches Patentamt
European Patent Office
Office européen des brevets

(11) Veröffentlichungsnummer: **0 089 576**
**B1**

(12) # EUROPÄISCHE PATENTSCHRIFT

(45) Veröffentlichungstag der Patentschrift: **10.07.85**

(51) Int. Cl.[4]: **C 07 C 69/18**, C 07 C 67/05, C 07 C 67/29

(21) Anmeldenummer: **83102404.7**

(22) Anmeldetag: **11.03.83**

(54) **1,1-Diacyloxy-3-alkyl-4-hydroxy-2-butene, ihre Herstellung und Verwendung.**

(30) Priorität: **19.03.82 DE 3210084**

(43) Veröffentlichungstag der Anmeldung: **28.09.83 Patentblatt 83/39**

(45) Bekanntmachung des Hinweises auf die Patenterteilung: **10.07.85 Patentblatt 85/28**

(84) Benannte Vertragsstaaten: **BE CH DE FR GB IT LI NL**

(56) Entgegenhaltungen:
**EP - A - 0 005 452**
**EP - A - 0 010 656**
**EP - A - 0 068 372**
**DE - A - 2 842 238**

(73) Patentinhaber: **BASF Aktiengesellschaft, Carl-Bosch-Strasse 38, D-6700 Ludwigshafen (DE)**

(72) Erfinder: **Weitz, Hans-Martin, Dr., Auf dem Koeppel 40, D-6702 Bad Duerkheim (DE)**
Erfinder: **Fischer, Rolf, Dr., Bergstrasse 98, D-6900 Heidelberg (DE)**

EP 0 089 576 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue 1,1-Diacyloxy-3-alkyl-4-hydroxy-2-butene, ein Verfahren zu ihrer Herstellung durch Umsetzung von 1-Acyloxy-3-alkyl-1,3-butadienen mit Carbonsäuren und Sauerstoff sowie ein Verfahren zur Herstellung von 2-Alkyl-4,4-diacyloxy-2-butenalen durch Oxidation der neuen 1,1-Diacyloxy-3-alkyl-4-hydroxy-2-butene.

Es ist aus der DE-OS 2 842 238 bekannt, daß bei der Umsetzung von 1-Acyloxy-3-alkyl-1,3-butadienen mit Carbonsäuren und Sauerstoff in Gegenwart von Palladium oder Platin enthaltenden Katalysatoren 3-Alkyl-1,1,4-triacyloxy-2-butene entstehen, die sich zu 3-Alkyl-4-acyloxy-2-butenalen verseifen lassen.

Es wurde nun gefunden, daß man neue 1,1-Diacyloxy-3-alkyl-4-hydroxy-2-butene der Formel

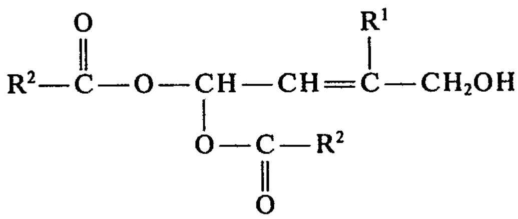

(I)

in der $R^1$ einen Alkylrest mit 1 bis 5 Kohlenstoffatomen und $R^2$ ein Wasserstoffatom, einen Alkylrest mit 1 bis 5 Kohlenstoffatomen, einen cycloaliphatischen Rest mit 5 bis 7 Kohlenstoffatomen oder einen aromatischen Rest bedeuten, erhält, wenn man 1-Acyloxy-3-alkyl-1,3-butadiene der Formel

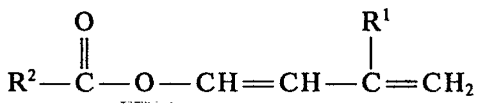

(III)

in der $R^1$ und $R^2$ die obengenannte Bedeutung haben, in Gegenwart von Sauerstoff oder von Sauerstoff abgebenden Verbindungen und in Abwesenheit von Palladium oder Platin enthaltenden Katalysatoren mit Carbonsäuren der Formel $R^2-COOH$, in der $R^2$ die obengenannte Bedeutung hat, umsetzt.

Von den Verbindungen der Formel I sind die 1,1-Diacyloxy-3-alkyl-4-hydroxy-2-butene der Formel

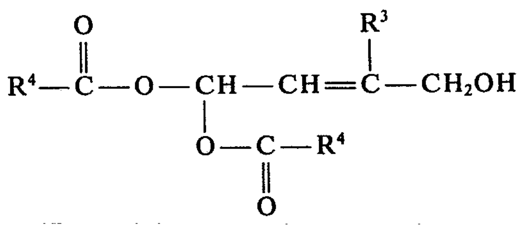

(II)

in der $R^3$ einen Alkylrest mit 1 bis 3 Kohlenstoffatomen und $R^4$ ein Wasserstoffatom oder einen Alkylrest mit 1 bis 3 Kohlenstoffatomen bedeutet, von besonderem technischen Interesse. Besonders bevorzugt ist das 1,1-Diacetoxy-3-methyl-4-hydroxy-2-buten.

In den Ausgangsstoffen der Formel III steht $R^1$ für einen Alkylrest mit 1 bis 5, vorzugsweise 1 bis 3 Kohlenstoffatomen. $R^2$ bedeutet ein Wasserstoffatom, einen Alkylrest mit 1 bis 5, vorzugsweise 1 bis 3 Kohlenstoffatomen, einen cycloaliphatischen Rest, z. B. einen Cyclopentyl-, Cyclohexyl- oder Cycloheptylrest oder einen aromatischen Rest, z. B. einen Phenylrest, der durch Alkylreste oder Halogenatome substituiert sein kann. Alkylreste sind z. B. Methyl-, Ethyl-, n-Propyl-, i-Propyl-, n-Butyl-, i-Butyl-, tert.-Butyl-, n-Pentylreste.

Die als Ausgangsstoffe zur Herstellung der neuen 1,1-Diacyloxy-3-alkyl-4-hydroxy-2-butene zu verwendenden a-Acyloxy-3-alkyl-1,3-butadiene sind durch Acylierung von $\alpha,\beta$-ungesättigten Aldehyden mit Säureanhydriden, Säurehalogeniden, Ketenen oder Enolestern zugänglich (Houben-Weyl, Methoden der organischen Chemie, 4. Auflage, Band V, 1c, Seiten 184 bis 192). So ist 1-Acetoxy-3-methyl-1,3-butadien z. B. aus 3-Methylcrotonaldehyd herstellbar.

Beispielsweise seien die folgenden Verbindungen der Formel III genannt:
3-Methyl-, 3-Ethyl-, 3-n-Propyl-, 3-Butyl-, 3-Pentyl-1-acetoxy-1,3-butadien, 3-Methyl-1-propionyloxy-1,3-butadien, 3-Methyl-1-cyclohexyloxy-1,3-butadien und 3-Methyl-1-benzoyloxy-1,3-butadien.

Als Carbonsäuren der Formel $R^2COOH$ kommen z. B. Ameisensäure, Essigsäure, Propionsäure, Buttersäure, Valeriansäure, Caprinsäure, Laurinsäure, Ölsäure, Palmitinsäure, Cyclohexancarbonsäure, Benzoesäure oder Phenylessigsäure in Betracht. Essigsäure ist aus wirtschaftlichen Gründen bevorzugt. Die Carbonsäure wird im allgemeinen im Überschuß, z. B. in der 1 bis 80molaren Menge, bezogen auf das eingesetzte 1,3-Dien III angewandt.

Sauerstoff kann in Form von reinem Sauerstoff oder im Gemisch mit anderen Gasen, wie Stickstoff z. B. in Form von Luft oder mit anderen inerten Gasen, wie Kohlendioxid verwendet werden. Als

Sauerstoff abgebende Verbindungen sind z. B. solche geeignet, die für die Epoxidierung von Olefinen verwendet werden, wie Wasserstoffperoxid, Persäuren, Perameisensäure, Peressigsäure, Perpropionsäure, Perbenzoesäure, Per-n-buttersäure, Per-iso-buttersäure und organische Hydroperoxide, wie tert.-Butylhydroperoxid oder Cumolhydroperoxid. Verbindungen dieser Art sind z. B. in Ullmanns Encyclopädie der Technischen Chemie, 4. Auflage, Band 10, Seiten 563 bis 567 genannt.

Das erfindungsgemäße Verfahren sei am Beispiel der Umsetzung von 1-Acetoxy-3-methyl-1,3-butadien mit Peressigsäure in Essigsäure zu 1,1-Diacetoxy-3-methyl-4-hydroxy-2-buten durch die folgenden Formeln erläutert:

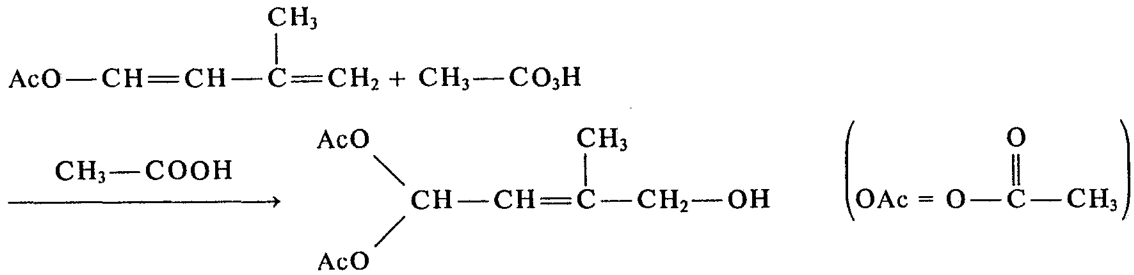


Im einzelnen wird das Verfahren z. B. so durchgeführt, daß man pro Mol 1,3-Dien der Formel III 1 bis 80 Mol, insbesondere 1,5 bis 60 Mol der Carbonsäure und 0,5 bis 5 Mol, insbesondere 1 bis 1,5 Mol an Sauerstoff oder der Sauerstoff abgebenden Verbindung anwendet. Man arbeitet zweckmäßig bei Temperaturen zwischen 0 und 200° C, insbesondere bei 20 bis 120° C und wendet z. B. Sauerstoffdrucke von 1 bis 100, insbesondere 1 bis 20 bar an.

Das Verfahren kann chargenweise oder kontinuierlich, drucklos oder unter Druck durchgeführt werden. Unumgesetzte 1,3-Diene (III) können gegebenenfalls nach der Umsetzung destillativ von den entstandenen Wertprodukten abgetrennt und erneut für die erfindungsgemäße Umsetzung verwendet werden.

Gewöhnlich arbeitet man entweder in einem Überschuß der Carbonsäure als Lösungsmittel oder mit geringeren Mengen an Carbonsäure und in Gegenwart von unter den Reaktionsbedingungen inerten Lösungsmitteln. Als Lösungsmittel dieser Art kommen z. B. Carbonsäureester, wie Essigsäuremethylester, chlorierte Kohlenwasserstoffe, wie Methylenchlorid, Chloroform, Tetrachlorkohlenstoff oder 1,2-Dichlorethan, Kohlenwasserstoffe, wie Alkane, Benzol und alkylierte Benzole, Ether, wie Diethylether, Tetrahydrofuran oder Dioxan in Betracht. Pro Mol Ausgangsverbindung (III) verwendet man zweckmäßigerweise 0,1 bis 80 Mol, insbesondere 2 bis 60 Mol, des unter den Reaktionsbedingungen inerten Lösungsmittels.

Die Umsetzung kann man bei diskontinuierlicher Arbeitsweise, z. B. wie folgt vornehmen: Einer Lösung des 1,3-Diens (III) in der Carbonsäure, die gegebenenfalls noch das Lösungsmittel enthält, werden bei der Reaktionstemperatur und dem Reaktionsdruck Sauerstoff oder die Sauerstoff abgebende Verbindung zugeführt. Nach beendeter Zugabe wird gegebenenfalls noch nachgerührt. Durch das auf Raumtemperatur abgekühlte Reaktionsgemisch wird gegebenenfalls Stickstoff geleitet. Anschließend wird nach dem Abdestillieren des Lösungsmittels und/oder der Carbonsäure fraktioniert destilliert. Dabei werden gegebenenfalls nicht umgesetzte Ausgangsverbindungen von den gewünschten Produkten abgetrennt.

Die erfindungsgemäßen 1,1-Diacyloxy-3-alkyl-4-hydroxy-2-butene sind wertvolle Zwischenprodukte, z. B. für die Herstellung von Terpenen. Sie werden für die Herstellung von 3-Alkyl-4-acyloxy-2-butenalen oder 2-Alkyl-4,4-diacyloxy-2-butenalen verwendet. Die 2-Alkyl-4,4-diacyloxy-2-butenale, die wertvolle Zwischenprodukte, z. B. für die Herstellung von Terpenen darstellen, erhält man durch Oxidation der neuen 1,1-Diacyloxy-3-alkyl-4-hydroxy-2-butene. Beispielsweise führt man die Oxidation durch, indem man die in einem Lösungsmittel gelösten 1,1-Diacyloxy-3-alkyl-4-hydroxy-2-butene mit Chromsäure enthaltenden Anionenaustauschern erhitzt (siehe Beispiel 2).

Zur Herstellung der 3-Alkyl-4-acyloxy-2-butenale werden die 1,1-Diacyloxy-4-hydroxy-2-butene durch Behandlung mit acylierenden Mitteln in die 3-Alkyl-1,1,4-triacyloxy-2-butene übergeführt, welche dann nach dem in der DE-OS 2 842 238 beschriebenen Verfahren zu den 2-Alkyl-4-acyloxy-2-butenaler hydrolysiert werden. Das auf diese Weise erhältliche 3-Methyl-4-acetoxy-2-butenal (siehe Beispiel 3) ist ein gesuchtes Zwischenprodukt für die Herstellung von Terpenen.

## Beispiel 1

### Herstellung von 1,1-Diacetoxy-3-methyl-4-hydroxy-2-buten durch Umsetzung von 1-Acetoxy-3-methyl-1,3-butadien mit Essigsäure und Peressigsäure

Zu einer Lösung von 72 g 1-Acetoxy-3-methyl-1,3-butadien in 100 g Eisessig wurden bei 30 bis 35°C unter Rühren und Kühlung innerhalb von einer Stunde 420 g Peressigsäure in Eisessig (12,7 Gew.-%, hergestellt nach Houben-Weyl, 4. Auflage, Methoden der organischen Chemie, Band 8, Seite 41) gegeben. Man rührte 18 Stunden bei Raumtemperatur. Peroxid konnte nach dieser Zeit nicht mehr nachgewiesen werden. Aus dem Reaktionsaustrag wurden Essigsäure und Niedrigsieder am Rotationsverdampfer zuerst bei 45°C/20 mbar und dann bei 100°C/0,2 mbar abgezogen. Man erhielt 96,1 g eines flüssigen Rückstands, aus dem durch Kugelrohrdestillation (150°C, 0,07 mbar) 74,8 g 1,1-Diacetoxy-3-methyl-4-hydroxy-2-buten, $n_D^{20}=1,4610$ erhalten wurden (65%, bezogen auf eingesetztes 1-Acetoxy-3-methyl-1,3-butadien).

$^1$H-NMR-Spektrum (Lösungsmittel $CDCl_3$, TMS als innerer Standard):
$\delta=1,80$ (breites s, 3H), 2,08 (s, 6H), 3,40 (s, 1H),

$$\left.\begin{matrix}4,02 & (s,\\ 4,20 & s,\end{matrix}\right\} 2H), \quad 5,55 \; (m, J=8\,Hz, 1H),$$

7,40 (d, J = 8 Hz, 1H).

## Beispiel 2

### Verwendung von 1,1-Diacetoxy-3-methyl-4-hydroxy-2-buten für die Herstellung von 2-Methyl-4,4-diacetoxy-2-butenal

Eine Lösung von 14,4 g 1,1-Diacetoxy-3-methyl-4-hydroxy-2-buten in 400 $cm^3$ Benzol wurde 10 Stunden in Gegenwart von 200 g des im Handel unter der Bezeichnung ®Amberlyst A-26 erhältlichen Anionenaustauscher unter Rückfluß erhitzt. Nach dem Abfiltrieren des Anionenaustauschers und Eindampfen der benzolischen Lösung durch Kugelrohrdestillation (90 bis 130°C/0,06 mbar) wurden 5,9 g 2-Methyl-4,4-diacetoxy-2-butenal, $n_D^{20}=1,4578$ (41%, bezogen auf eingesetztes 1,1-Diacetoxy-3-methyl-4-hydroxy-2-buten) erhalten. ®Amberlyst A-26 ist ein Anionenaustauscher, der pro Gramm 2,2 mMol $CrO_3$ enthält.

## Beispiel 3

### Verwendung von 1,1-Diacetoxy-3-methyl-4-hydroxy-2-buten für die Herstellung von 3-Methyl-4-acetoxy-2-butenal

#### a) Acetylierung zu 3-Methyl-1,1,4-triacetoxy-2-buten

7,5 g des nach Beispiel 1 hergestellten 1,1-Diacetoxy-3-methyl-4-hydroxy-2-butens wurden in 15 $cm^3$ Pyridin gelöst. Nach Zugabe von 5,2 g Acetylchlorid wurde die Lösung 2 Stunden bei 35°C gerührt. Das Reaktionsgemisch wurde in 150 $cm^3$ Ether aufgenommen. Die Etherphase wurde mit Wasser ausgeschüttelt und über Magnesiumsulfat getrocknet. Nach Abziehen des Ethers am Rotationsverdampfer erhielt man durch Kugelrohrdestillation (140°C, 0,06 mbar) 7,5 g 3-Methyl-1,1,4-triacetoxy-2-buten, $n_D^{20}=1,4505$ (82%, bezogen auf eingesetztes 1,1-Diacetoxy-3-methyl-4-hydroxy-2-buten).

#### b) Hydrolyse von 3-Methyl-1,1,4-triacetoxy-2-buten

5,4 g des nach diesem Beispiel, Absatz (a) hergestellten 3-Methyl-1,1,4-triacetoxy-2-butens wurden mit 50 g Essigsäure und 4,4 g Wasser versetzt. Das Gemisch wurde 2 Stunden auf 100°C erhitzt. Nach Abziehen von Essigsäure und Wasser am Rotationsverdampfer wurden durch Kugelrohrdestillation (75°C, 0,1 mbar) 2,3 g 3-Methyl-4-acetoxy-2-butenal vom Brechungsindex $n_D^{20}=1,4720$ (73%, bezogen auf eingesetztes 3-Methyl-1,1,4-triacetoxy-2-buten) erhalten.

**Patentansprüche**

1. 1,1-Diacyloxy-3-alkyl-4-hydroxy-2-butene der Formel

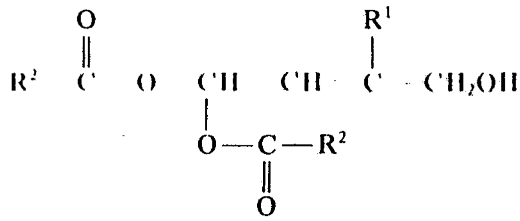

(I)

in der $R^1$ einen Alkylrest mit 1 bis 5 Kohlenstoffatomen und $R^2$ ein Wasserstoffatom, einen Alkylrest mit 1 bis 5 Kohlenstoffatomen, einen cycloaliphatischen Rest mit 5 bis 7 Kohlenstoffatomen oder einen aromatischen Rest bedeuten.

2. 1,1-Diacyloxy-3-alkyl-4-hydroxy-2-butene der Formel

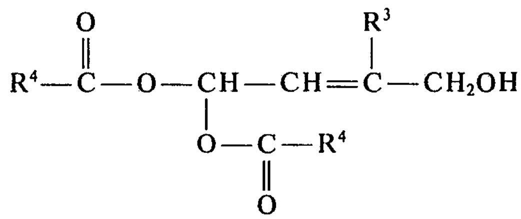

(II)

in der $R^3$ einen Alkylrest mit 1 bis 3 Kohlenstoffatomen und $R^4$ ein Wasserstoffatom oder einen Alkylrest mit 1 bis 3 Kohlenstoffatomen bedeutet.

3. 1,1-Diacetoxy-3-methyl-4-hydroxy-2-buten.

4. Verfahren zur Herstellung der 1,1-Diacyloxy-3-alkyl-4-hydroxy-2-butene gemäß Anspruch 1, dadurch gekennzeichnet, daß man 1-Acyloxy-3-alkyl-1,3-butadiene der Formel

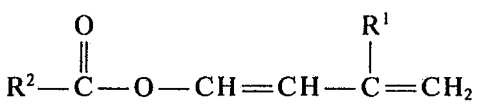

(III)

in der $R^1$ und $R^2$ die in Anspruch 1 genannte Bedeutung haben, in Gegenwart von Sauerstoff oder von Sauerstoff abgebenden Verbindungen und in Abwesenheit von Palladium oder Platin enthaltenden Katalysatoren mit Carbonsäuren der Formel $R^2-COOH$, in der $R^2$ die obengenannte Bedeutung hat, umsetzt.

5. Verfahren zur Herstellung von 2-Alkyl-4,4-diacyloxy-2-butenalen, dadurch gekennzeichnet, daß man 1,1-Diacyloxy-3-alkyl-4-hydroxy-2-butene gemäß Anspruch 1 oxidiert.

**Claims**

1. A 1,1-diacyloxy-3-alkyl-4-hydroxy-2-butene of the formula

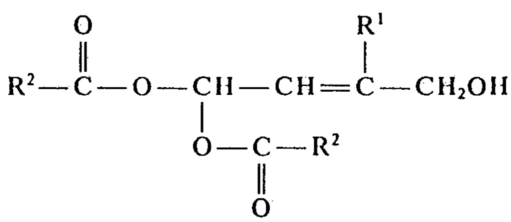

(I)

where $R^1$ is alkyl of 1 to 5 carbon atoms, and $R^2$ is hydrogen, alkyl of 1 to 5 carbon atoms, a cycloaliphatic radical of 5 to 7 carbon atoms or an aromatic radical.

2. A 1,1-diacyloxy-3-alkyl-4-hydroxy-2-butene of the formula

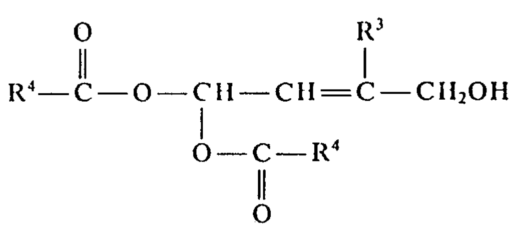

(II)

where $R^3$ is alkyl of 1 to 3 carbon atoms, and $R^4$ is hydrogen, or alkyl of 1 to 3 carbon atoms.

3. 1,1-Diacetoxy-3-methyl-4-hydroxy-2-butene.

4. A process for the preparation of a 1,1-diacyloxy-3-alkyl-4-hydroxy-2-butene as claimed in claim 1, wherein a 1-acyloxy-3-alkyl-1,3-butadiene of the formula

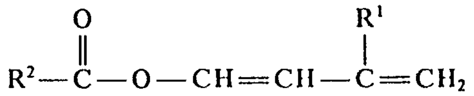

(III)

where $R^1$ and $R^2$ have the meanings given in claim 1, is reacted with a carboxylic acid of the formula $R^2-COOH$, where $R^2$ has the above meanings, in the presence of oxygen or an oxygen-liberating compound, and in the absence of a palladium- or platinum-containing catalyst.

5. A process for the preparation of a 2-alkyl-4,4-diacyloxy-2-butenal, wherein a 1,1-diacyloxy-3-alkyl-4-hydroxy-2-butene as claimed in claim 1 is oxidized.

**Revendications**

1. 1,1-Diacyloxy-3-alcoyl-4-hydroxy-2-butènes de la formule

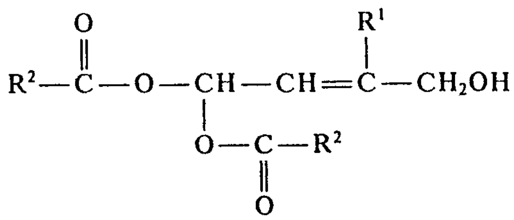

(I)

dans laquelle $R^1$ désigne un radical alcoyle en $C_1$ à $C_5$ et $R^2$ un atome d'hydrogène, un radical alcoyle en $C_1$ à $C_5$, un groupe cycloaliphatique en $C_5$ à $C_7$ ou un groupe aromatique.

2. 1,1-Diacyloxy-3-alcoyl-4-hydroxy-2-butènes de la formule

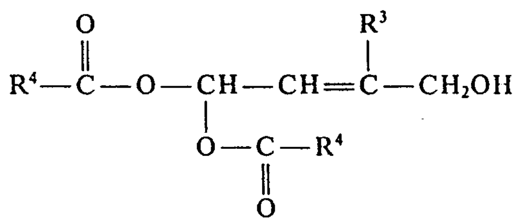

(II)

dans laquelle $R^3$ désigne un radical alcoyle en $C_1$ à $C_3$ et $R^4$ un atome d'hydrogène ou un radical alcoyle en $C_1$ à $C_3$.

3. Le 1,1-diacétoxy-3-méthyl-4-hydroxy-2-butène.

4. Procédé de préparation des 1,1-diacyloxy-3-alcoyl-4-hydroxy-2-butènes selon la revendication 1, caractérisé en ce que l'on fait réagir des 1-acyloxy-3-alcoyl-1,3-butadiènes de la formule

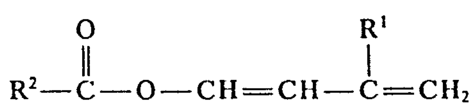

(III)

dans laquelle $R^1$ et $R^2$ possèdent la signification définie dans la revendication 1, en présence d'oxygène ou de composés libérant de l'oxygène et en l'absence de catalyseurs au palladium ou au platine, avec des acides carboxyliques de la formule

$R^2-COOH$

dans laquelle $R^2$ possède la signification définie plus haut.

5. Procédé de préparation de 2-alcoyl-4,4-diacyloxy-2-buténals, caractérisé en ce que l'on oxyde des 1,1-diacyloxy-3-alcoyl-4-hydroxy-2-butènes selon la revendication 1.